# EUROPEAN PATENT APPLICATION

(11) **EP 2 945 086 A2**
(43) Date of publication of application: **18.11.2015**
(21) Application number: 15166809.2
(22) Date of filing: 07.05.2015
(51) Int. Cl.: G06F 19/00, G06Q 30/00

(54) **IMAGE MANAGEMENT SYSTEM AND IMAGE MANAGEMENT METHOD**

(30) Priority: 16.05.2014 JP 2014102504
(71) Applicant: Ricoh Company, Ltd., Tokyo 143-8555 (JP)
(72) Inventor: Ninomiya, Michiaki, Tokyo, 143-8555 (JP)
(74) Representative: Leeming, John Gerard

(57) **Abstract**

An image management system including an image capturing apparatus (200) and an information processing apparatus (100) is provided. The image capturing apparatus includes a recognition unit (202) that recognizes identification information represented by an optically readable code, an acquiring unit (201) that acquires image data of an imaging object, a recording unit (203) that records the identification information recognized by the recognition unit and the image data acquired by the acquiring unit in association with each other, and a transmitting unit (205) configured to transmit the identification information and the image data recorded by the recording unit to the information processing apparatus. The information processing apparatus includes a storage unit (101) that stores the image data based on the identification information received from the image capturing apparatus.

## Description

The present invention relates to an image management system and an image management method.

With the growing popularity of digital cameras, systems for managing images that are acquired by a digital camera and uploaded to a server via a network are becoming common. In such systems, a large number of images that have been uploaded by a user may be grouped and managed according to date, an event, and the like (see e.g., Japanese Laid-Open Patent Publication No. 2012-049906).

Japanese Laid-Open Patent Publication No. 2012-159996 discloses an image capturing apparatus that is configured to display a barcode on a display after an image has been uploaded to a server via wireless communication, wherein a user is prompted to capture an image of the barcode using a mobile phone so that the user may be guided to an image editing site on the server.

However, according to conventional techniques for managing image data of a large number of captured images, one or more sets of image data relating to one imaging object are collectively managed in a batch and rather cumbersome operations were required to manage such image data such as checking each set of image data, manually dividing folders, and assigning tags, for example.

In view of the above problems of the related art, an aspect of the present invention relates to efficiently managing image data relating to one imaging object.

According to one embodiment of the present invention, an image management system including an image capturing apparatus and an information processing apparatus is provided. The image capturing apparatus includes a recognition unit that recognizes identification information represented by an optically readable code, an acquiring unit that acquires image data of an imaging object, a recording unit that records the identification information recognized by the recognition unit and the image data acquired by the acquiring unit in association with each other, and a transmitting unit configured to transmit the identification information and the image data recorded by the recording unit to the information processing apparatus. The information processing apparatus includes a storage unit that stores the image data based on the identification information received from the image capturing apparatus.

In the accompanying drawings:
FIG. 1 schematically illustrates an image management system according to an embodiment of the present invention;
FIG. 2 is an external view of an image capturing apparatus according to an embodiment of the present invention;
FIG. 3 illustrates a hardware configuration of an information processing apparatus according to an embodiment of the present invention;
FIG. 4 illustrates a hardware configuration of the image capturing apparatus according to an embodiment of the present invention;
FIG. 5 illustrates a hardware configuration of an image forming apparatus according to an embodiment of the present invention;
FIG. 6 illustrates a functional configuration of the image management system according to an embodiment of the present invention;
FIG. 7 illustrates an example of an output image output by the image forming apparatus;
FIG. 8 illustrates an exemplary configuration of identification information;
FIG. 9 illustrates an example of a folder storing image data;
FIG. 10 is a flowchart illustrating a barcode output process;
FIG. 11 is a flowchart illustrating a barcode recognition and image data recording process;
FIG. 12 is a flowchart illustrating an image data storage process;
FIG. 13 is a sequence chart illustrating exemplary operations of the image management system;
FIG. 14 illustrates an exemplary application of the image management system;
FIG. 15 illustrates another exemplary application of the image management system;
FIG. 16 illustrates another exemplary application of the image management system; and
FIG. 17 illustrates another exemplary application of the image management system.

### DESCRIPTION OF THE PREFERRED EMBODIMENTS

In the following, embodiments of the present invention are described with reference to the accompanying drawings.

### «System»

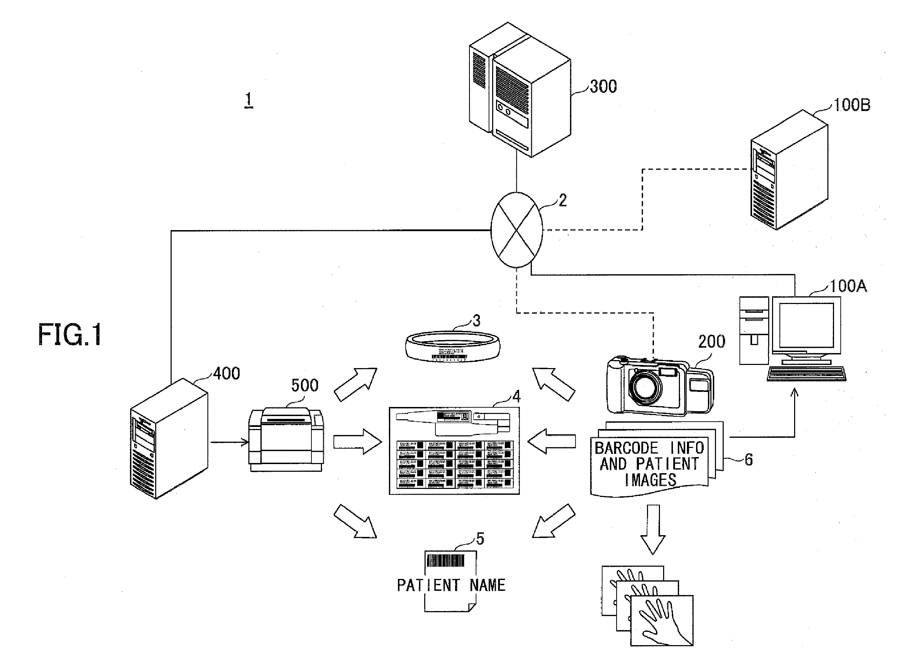

FIG. 1 schematically illustrates an image management system 1 according to an embodiment of the present invention. The image management system 1 includes information processing apparatuses 100A and 100B, an image capturing apparatus 200, a management system 300, an information processing apparatus 400, and an image forming apparatus 500 that are interconnected via at least one network 2 such as a LAN (local area network) and/or the Internet.

In the present embodiment, an application of the image management system 1 to a medical care system for managing patient data is described as an example.

The management system 300 may be an HIS (Hospital Information System) that is used in a hospital, for example. The HIS includes PACS (Picture Archiving and Communication Systems) for managing image data of each patient. That is, the management system 300 is capable of managing image data of a patient obtained through examination and diagnosis of the patient and outputting the image data in response to a request from a person in charge, for example. Also, the management system 300 may manage patient information including an identification number of the patient (patient number), the name of the patient (patient name), a photo of the patient (patient photo), and the date of birth of the patient, for example.

The information processing apparatus 400 acquires the patient information from the management system 300 via the network 2. The information processing apparatus 400 may generate a barcode corresponding to the patient number and control the image forming apparatus 500 to output the barcode and the patient information in different formats according to the intended use, for example. Note that other types of optically readable graphics or code such as a QA code (registered trademark) or a three-dimensional color barcode may be used instead of the barcode used in the present embodiment.

The image forming apparatus 500 outputs the barcode and the patient information according to control by the information processing apparatus 400. For example, the image forming apparatus 500 may output the barcode and the patient information onto a label 3 to be attached to an accessory (e.g., wristband) that is secured to the patient's body (e.g., arm). Also, the image forming apparatus 500 may output the barcode and the patient information onto one sheet 4 including a prefabricated wristband and plural labels, for example. Also, the image forming apparatus 500 may output the barcode and the patient information together with medical records and medical examination charts 5, for example.

The image capturing apparatus 200 is capable of recognizing patient information such as a patient number from a barcode or some other form of graphics. Also, once the image capturing apparatus 200 recognizes the patient number from a barcode of a patient, the image capturing apparatus 200 may automatically associate image data of an affected area of the patient obtained thereafter with the corresponding patient number of the patient.

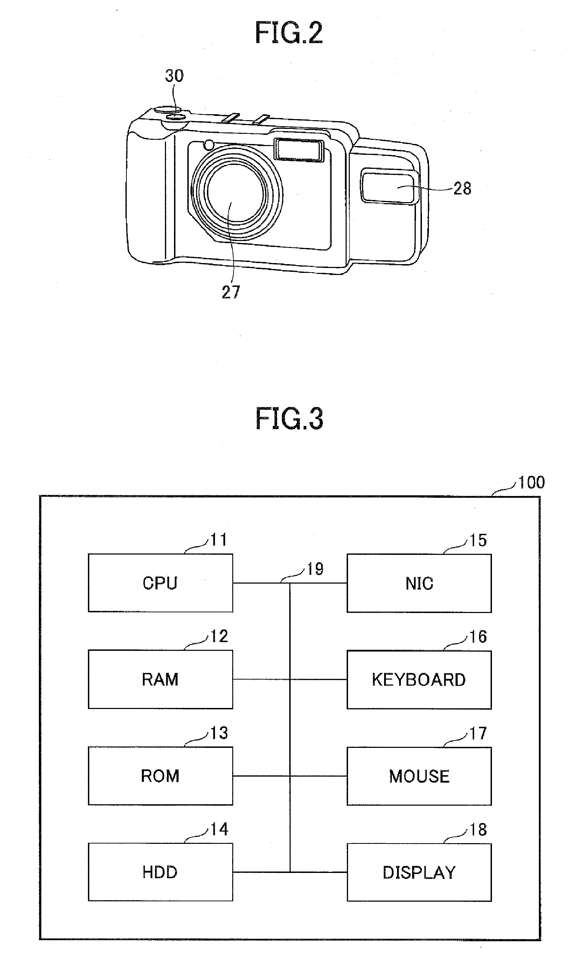

FIG. 2 illustrates a housing of the image capturing apparatus 200 that is used in the present embodiment. The image capturing apparatus 200 includes a CCD 227, an operation unit 30, and a barcode reading unit 28. The image capturing apparatus 200 uses the barcode reading unit 28 to recognize the patient number represented by a barcode.

The image capturing apparatus 200 may use a USB (Universal Serial Bus) cable or a wireless LAN to transmit the recognized patient information and image data to the information processing apparatuses 100A and 100B.

The information processing apparatus 100A acquires the patient information and image data from the image capturing apparatus 200 via a USB cable. The information processing apparatus 100A uses the acquired patient information to identify a folder in which the acquired image data is to be stored and stores the image data in the corresponding folder.

The information processing apparatus 100B acquires the patient information and image data from the image capturing apparatus 200 via a wireless LAN. The information processing apparatus 100B uses the acquired patient information to store the acquired image data in a corresponding folder in a manner similar to the above storage process operations of the information processing apparatus 100A. However, unlike the information processing apparatus 100A, the information processing apparatus 100B corresponds to a server on the Internet that is provided by a third party that is different from the owner of the image capturing apparatus 200.

The management system 300 is capable of acquiring the folders and the image data managed by the information processing apparatuses 100A and 100B. In this way, the management system 300 may acquire image data organized by the patient number.

Oftentimes, medical personnel are too busy to operate image capturing apparatuses and information processing apparatuses to associate image data with a patient number while they are engaging in medical practice. However, according to the image management system 1 of the present embodiment, the medical personnel may simply capture an image of the barcode of a patient and then capture an image of an affected area of the patient to associate the image data of the affected area with the corresponding patient. In this way, the burden on the medical personnel may be reduced, and errors in associating image data with a corresponding patient may be prevented.

### «Hardware Configuration»

In the following, hardware configurations of the information processing apparatuses 100A and 100B (hereinafter collectively referred to as "information processing apparatus 100"), the image capturing apparatus 200, and the image forming apparatus 500 are described with reference to FIGS. 3-5.

### «Information Processing Apparatus»

FIG. 3 illustrates a hardware configuration of the information processing apparatus 100 according to an embodiment of the present invention. The information processing apparatus 100 includes a CPU 11, a RAM 12, a ROM 13, and an HDD 14, a NIC (Network Interface Card) 15, a keyboard 16, a mouse 17, and a display 18.

The CPU 11 executes a program for controlling the operation of the information processing apparatus 100. The program is configured to control the operation of the information processing apparatus 100 to implement some or all of the functional features described below. The RAM 12 provides a work area for the CPU 11. The ROM 13 stores a system program for starting the information processing apparatus 100. The HDD 14 stores an operating system (OS) for managing the operation of the information processing apparatus 100 and data necessary for running the OS. The HDD 14 also stores image data acquired from the image capturing apparatus 200.

The NIC 15 is a communication interface for establishing connection with a LAN. The keyboard 16 and the mouse 17 are devices that receive inputs from an operator of the information processing apparatus 100. The display 18 is a device for presenting information to the operator of the information processing apparatus 100. The bus 19 establishes electrical connection between the above hardware components.

Note that in some embodiments, the information processing apparatus 100 may be a server that inputs/outputs information to another client connected via a network, for example. In this case, the information processing apparatus 100 does not have to include the keyboard 16, the mouse 17 and the display 18.

The information processing apparatus 100 may be configured by a single housing or may be configured by a plurality of housings. For example, the information processing apparatus 100 may use a storage area on a network as a data storage area instead of the HDD 14.

### «Image Capturing Apparatus»

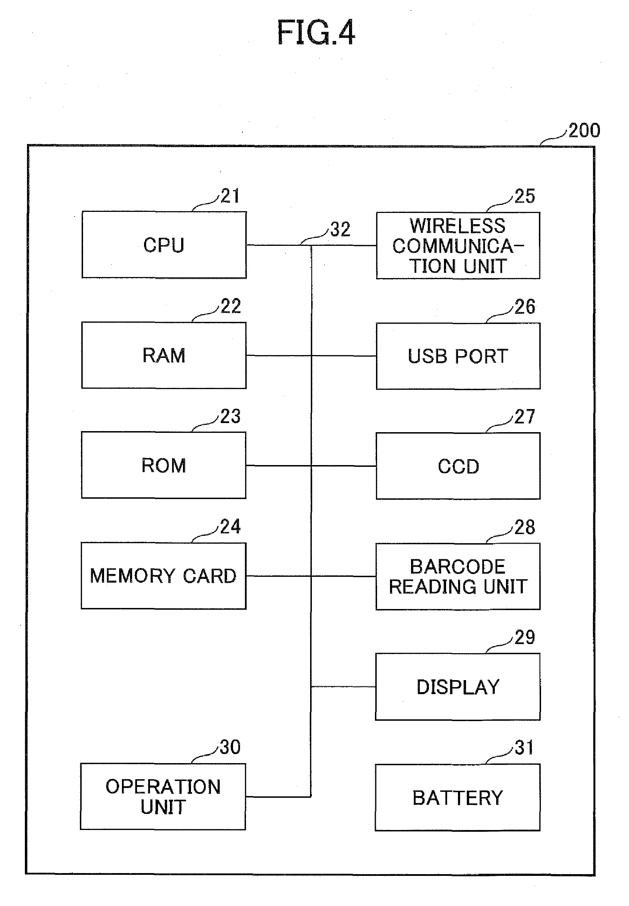

FIG. 4 illustrates a hardware configuration of the image capturing apparatus 200 according to an embodiment of the present invention. The image capturing apparatus 200 includes a CPU 21, a RAM 22, a ROM 23, a memory card 24, a wireless communication unit 25, a USB port 26, a CCD (Charge-Coupled Device) 27, a barcode reading unit 28, a display 29, an operation unit 30, and a battery 31.

The CPU 21 executes a program for controlling the operation of the image capturing apparatus 200. The program may be configured to control the operation of the image capturing apparatus 200 to implement some or all of the functional features described below. The RAM 22 provides a work area for the CPU 21. The ROM 23 stores a system program for operating the image capturing apparatus 200. The memory card 24 stores image data of captured images.

The wireless communication unit 25 is a wireless communication module for establishing connection with a wireless LAN. The USB port 26 is an interface for connecting a USB cable to establish connection with the information processing apparatus 100. The CCD 27 is a device that forms an image by converting light emitted from an imaging object into an electrical signal to generate image data. The barcode reading unit 28 is a device that includes a light source and a light sensor. The barcode reading unit 28 is capable of reading specific information such as patient information by reading bar and space patterns of a barcode.

The display 29 is a device for presenting information to the operator of the image capturing apparatus 200. The operation unit 30 is a device for receiving input from the operator of the image capturing apparatus 200. The battery 31 supplies power for driving the image capturing apparatus 200. The bus 32 establishes electrical connection between the above hardware components except for the battery 31.

### «Image Forming Apparatus»

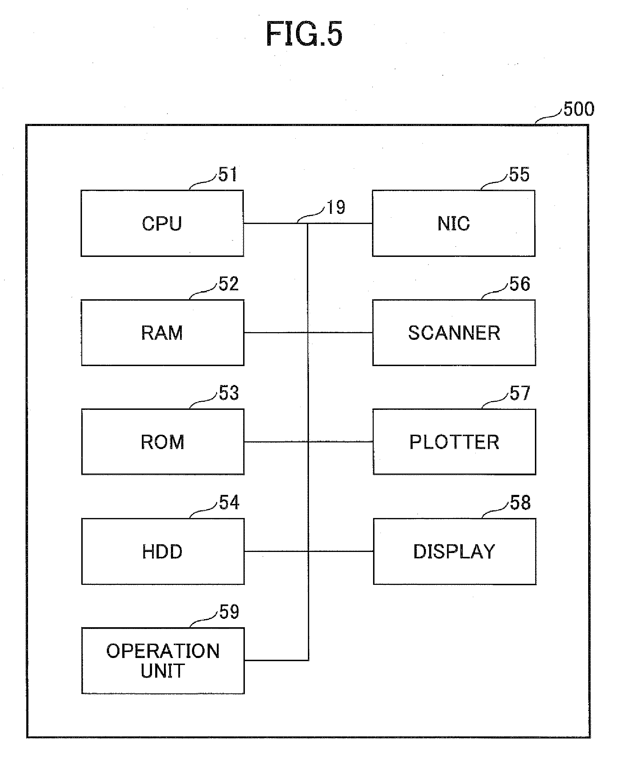

FIG. 5 illustrates a hardware configuration of the image forming apparatus 500 according to an embodiment of the present invention. The image forming apparatus 500 includes a CPU 51, a RAM 52, a ROM 53, an HDD 54, an NIC 55, a scanner 56, a plotter 57, a display 58, and an operation unit 59.

The CPU 51 executes a program for controlling the operation of the image forming apparatus 500. The program may be configured to control the operation of the image forming apparatus 500 to implement some or all of the functional features described below. The RAM 52 provides a work area for the CPU 51. The ROM 53 stores a system program for starting the image forming apparatus 500. The HDD 54 stores an OS for managing the operation of the image forming apparatus 500 and data necessary for running the OS.

The NIC 55 is a communication interface for establishing connection with a LAN. The scanner 56 irradiates light on a document placed on a platen from the platen side and converts the reflected light into an electrical signal with an image pickup device to digitize information of the document. The plotter 57 forms an image with toner or ink on the surface of a medium such as paper based on image data of a barcode and/or text data of the name of a patient and the date of birth of the patient, for example.

The operation unit 59 is a device for accepting an input from the operator of the image forming apparatus 500. The bus 60 establishes electrical connection between the above hardware components.

### «Function»

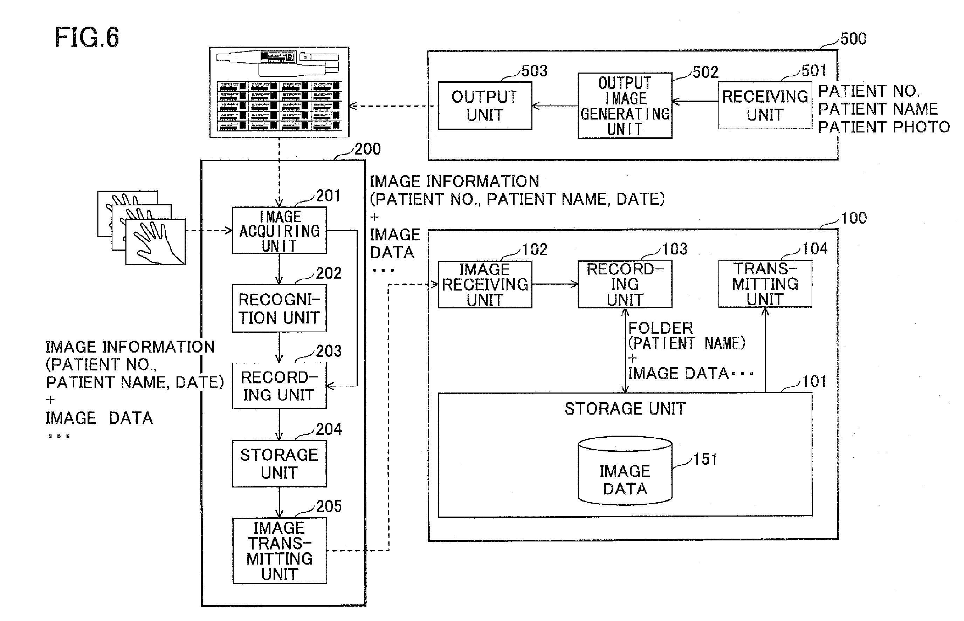

FIG. 6 is a block diagram illustrating a functional configuration of the image management system 1 according to an embodiment of the present invention. Note that FIG. 6 illustrates the functional configurations of the image forming apparatus 500, the image capturing apparatus 200, and the information processing apparatus 100 of the image management system 1 described above with reference to FIG. 1.

First, the image forming apparatus 500 according to an embodiment of the present invention includes a receiving unit 501, an output image generating unit 502, and an output unit 503.

The receiving unit 501 may be implemented by a process executed by the CPU 51 and the NIC 55 of FIG. 5, for example. The receiving unit 501 receives patient information including a patient number, a patient name, and a patient photo, for example, from the management system 300.

The output image generating unit 502 may be implemented by a process executed primarily by the CPU 51 of FIG. 5, for example. The output image generating unit 502 generates a barcode corresponding to the patient number received by the receiving unit 501 and generates an output image for printing including the generated barcode and some or all of the received patient information.

For example, the output image generating unit 502 may generate an output image including the barcode and patient information to be printed on a label 3, and the label 3 with the output image printed thereon may be attached to a wristband to be secured to the arm of a patient. Also, the image forming apparatus 500 may generate an output image including the barcode and patient information to be printed on one sheet 4 including a prefabricated wristband and a plurality of labels, for example. Also, the image forming apparatus 500 may generate an output image of medical records and medical examination charts 5 including the barcode and patient information, for example.

The output unit 503 may be implemented by a process executed by the plotter 57 of FIG. 5, for example. The output unit 503 prints out the output image generated by the output image generating unit 502 on a corresponding medium such as paper.

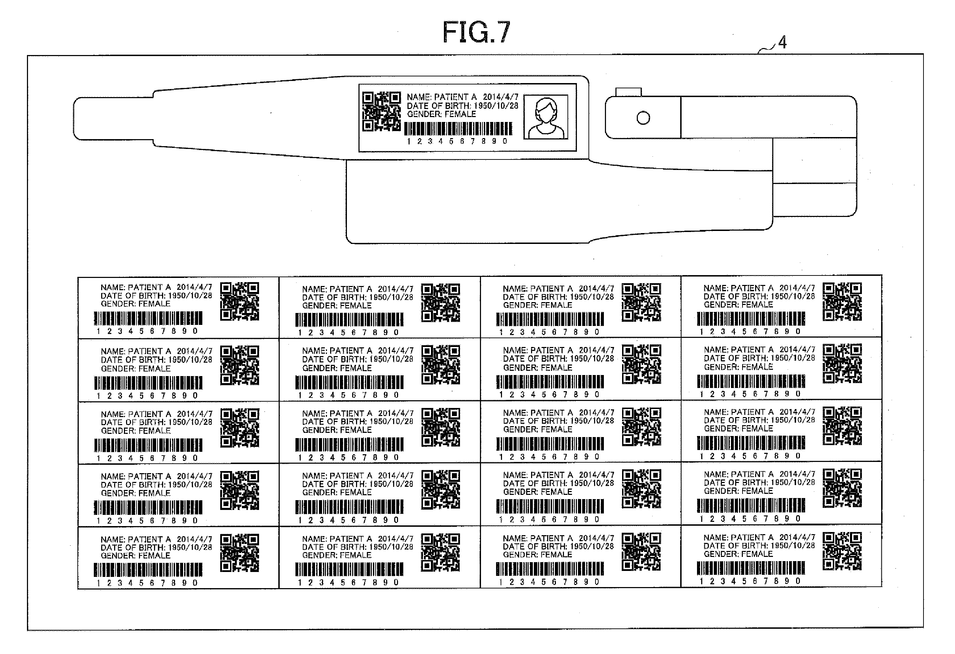

FIG. 7 illustrates an example in which the output unit 503 has printed out barcode and patient information onto one sheet 4 including a prefabricated wristband and a plurality of labels. Medical personnel may assemble and hand the prefabricated wristband to the patient and attach the labels on examination records and test containers of the patient in order to prevent erroneous association of test results with different patients, for example.

The image capturing apparatus 200 according to an embodiment of the present invention includes an image acquiring unit 201, a recognition unit 202, a recording unit 203, a storage unit 204, and an image transmitting unit 205.

The image acquiring unit 201 may be implemented by a process executed by the barcode reading unit 28 or the CCD 27 of FIG. 4, for example. The image acquiring unit 201 acquires an image of the barcode or an image of some or all of an affected area of a patient. In the case where the image acquiring unit 201 acquires an image of the barcode, the image acquiring unit 201 passes the acquired image to the recognition unit 202. On the other hand, in the case where the image acquiring unit 201 acquires an image of an affected area of a patient, the image acquiring unit 201 passes the acquired image to the recording unit 203.

The recognition unit 202 may be implemented by a process executed by the barcode reading unit 28 and/or the CPU 21 of FIG. 4, for example. The recognition unit 202 recognizes the corresponding patient number from the image of the barcode acquired by the image acquiring unit 201 and passes the recognized patient number to the recording unit 203.

Note that the barcode may be a one-dimensional bar code or a two-dimensional barcode as illustrated in FIG. 7. By recognizing the two-dimensional barcode, the recognition unit 202 may be able to recognize more structured and complicated information as compared with the case of recognizing the one-dimensional bar code, for example.

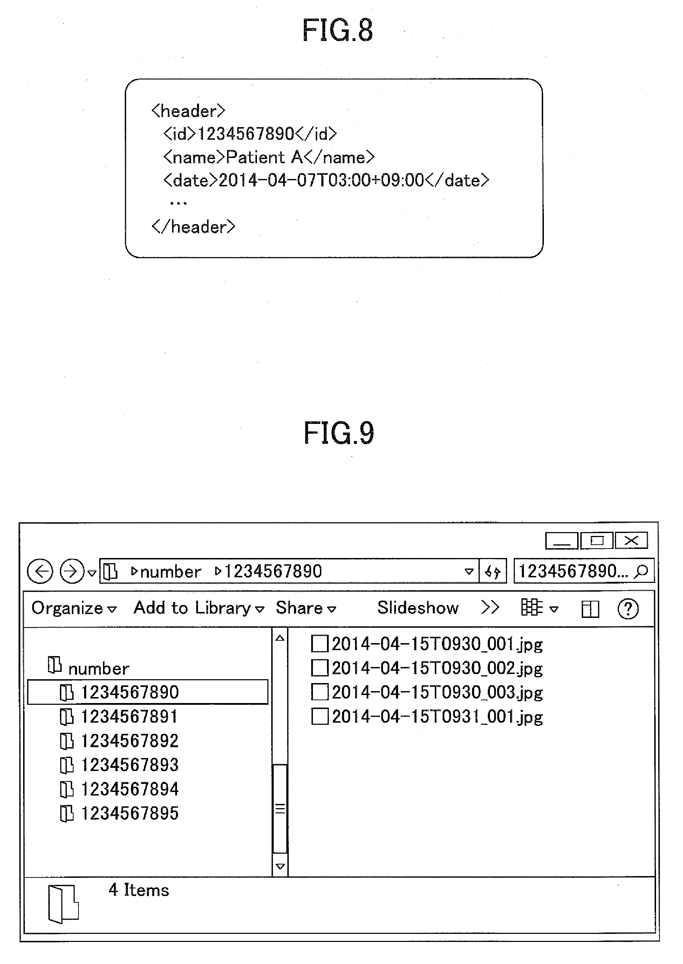

FIG. 8 illustrates an example of data in the XML (eXtensible Markup Language) format that may be obtained when the recognition unit 202 recognizes the two-dimensional barcode. In this case, the recognition unit 202 may recognize information items such as the patient name ("name") and the output time ("date"), for example, in addition to the patient number ("id").

The recording unit 203 may be implemented by a process executed by the CPU 21 of FIG. 4, for example. The recording unit 203 records the image of the patient acquired by the image acquiring unit 201 as image data in the storage unit 204. At this time, the recording unit 203 is able to record the patient number received from the recognition unit 202 in association with the image data.

Note that the recording unit 203 may record the image data in association with the patient number by any given method. For example, the recording unit 203 may create a text file containing the patient number and pair up the image data with the text file to associate the image data with the patient number. In another example, the recording unit 203 may record the patient number in a header of the image data to associate the image data with the patient number.

The storage unit 204 may be implemented by the memory card 24 of FIG. 4, for example. The storage unit 204 stores one or more combinations of the patient number and the image data.

The image transmitting unit 205 may be implemented by a process executed by the CPU 21 and the wireless communication unit 25 or the USB port 26 of FIG. 4, for example. The image transmitting unit 205 transmits a combination of the patient number and the image data stored in the storage unit 204 to the information processing apparatus 100.

The information processing apparatus 100 according to an embodiment of the present invention includes a storage unit 101, an image receiving unit 102, a recording unit 103, and a transmitting unit 104.

The storage unit 101 may be implemented by the HDD 14 of FIG. 3, for example. The storage unit 101 stores, for each folder created with respect to each patient number, image data 151 of an affected area of the corresponding patient that is identified by the patient number.

The image receiving unit 102 may be implemented by a process executed by the CPU 11 and the NIC 15 of FIG. 3, for example. The image receiving unit 102 receives a set of the patient number and the image data from the image capturing device 200.

The recording unit 103 may be implemented by a process executed by the CPU 11 of FIG. 3, for example. The recording unit 103 may create a folder in the storage unit 101 using the patient number received by the image receiving unit 102 and record the image data 151 that is paired up with the patient number in the folder. Also, in the case where the patient number is recorded in the header of the image data as described above, the recording unit 103 may read the header to obtain the patient number.

FIG. 9 illustrates an exemplary configuration of folders created in the storage unit 101 by the recording unit 103 and image data 151 stored in a folder. Note that FIG. 9 illustrates an example in which four sets of image data 151 are stored in association with the patient number "1234567890".

The transmitting unit 104 may be implemented by a process executed by the CPU 11 and the NIC 15 of FIG. 3, for example. The transmitting unit 104 transmits the image data 151 stored in the storage unit 101 to the management system 300.

### «Operation»

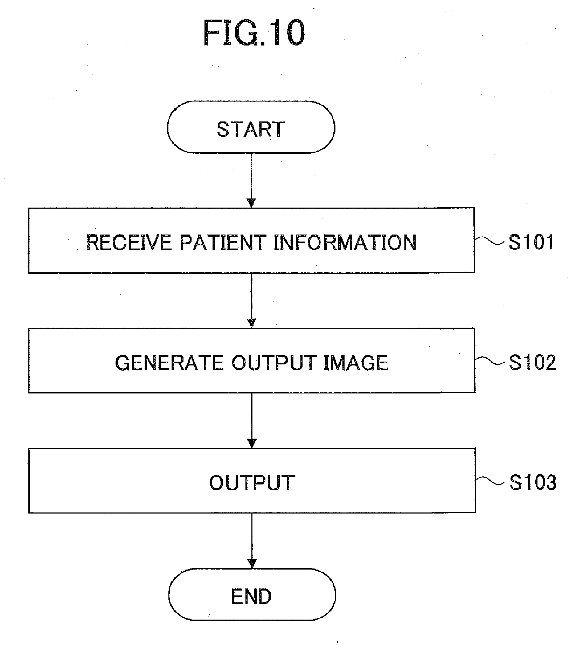

In the following, specific operations of the image management system 1 according to embodiments of the present invention are described with reference to FIGS. 10-13.

### «Barcode Output Process»

FIG. 10 is a flowchart illustrating a barcode output process executed by the image forming apparatus 500 according to an embodiment of the present invention.

First, the receiving unit 501 receives patient information including a patient number, a patient name, and a patient photo, for example, from the management system 300 (step S101). Then, the output image generating unit 502 generates a barcode corresponding to the patient number and generates an output image for printing including the barcode and some or all of the patient information received by the receiving unit 501 (step S102). For example, the output image generating unit 502 may generate a one-dimensional barcode representing the patient number and a two-dimensional barcode representing the patient number as well as the patient name and the date on which the barcode was generated, for example. The output image generating unit 502 may then generate an output image including the one-dimensional barcode and the two-dimensional barcode as illustrated in FIG. 7. The output unit 503 outputs (e.g. prints) the output image generated by the output image generating unit 502 onto the sheet 4 (step S103).

Medical personnel may hand the prefabricated wristband formed on the sheet 4 to the corresponding patient and attach the labels to examination records and test containers of the patient, for example. Also, in a case where the medical personnel has to capture an image of an affected area of the patient, the medical personnel may capture an image of the barcode before capturing the image of the affected area so that the image of the affected area may be properly associated with the patient without requiring any special operation.

### «Image Data Recording Process»

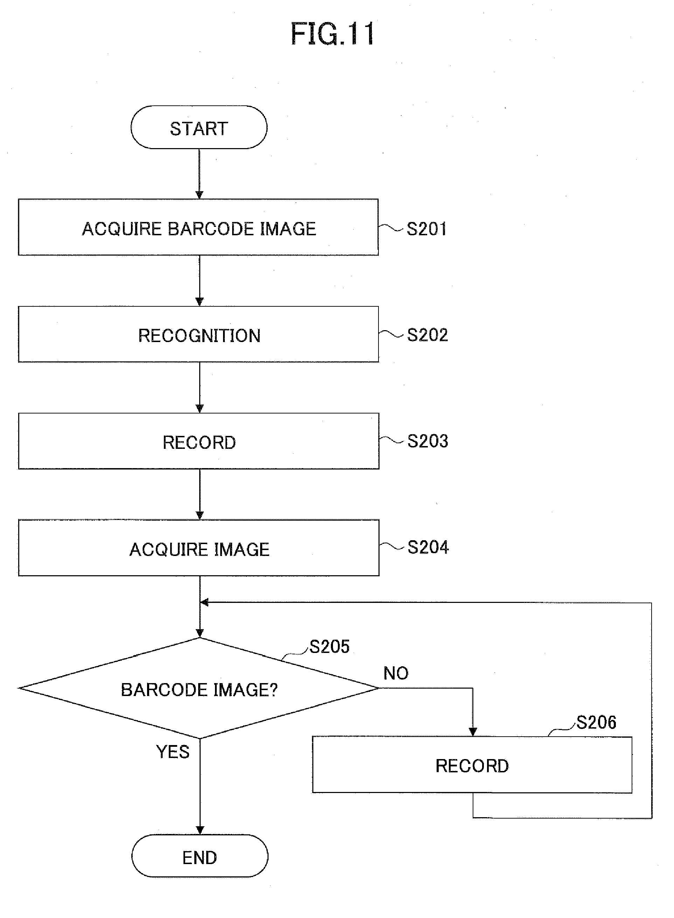

FIG. 11 is a flowchart illustrating a barcode recognition and image data recording process executed by the image capturing apparatus 200 according to an embodiment of the present invention. Note that the process illustrated in FIG. 11 may be performed with respect to each patient.

First, the image acquiring unit 201 acquires an image of a barcode in response to an operation of the operation unit 30 by an operator (step S201). In the present example, it is assumed that in this step, an image of the one-dimensional barcode of the barcodes illustrated in FIG. 7 is acquired by the image acquiring unit 201. The recognition unit 202 recognizes the corresponding patient number from the barcode image acquired by the image acquiring unit 201 (step S202). Then, the recording unit 203 records the patient number recognized by recognition unit 202 in the storage unit 204 (step S203).

Then, the image acquiring unit 201 acquires a barcode image or an image of an affected area of the patient in response to an operation of the operation unit 30 by the operator (step S204). The image acquiring unit 201 determines whether the acquired image corresponds to a barcode image (step S205). If the acquired image corresponds to a barcode image (YES in step S205), the present process is terminated. On the other hand, if the acquired image corresponds to an image of an affected area of the patient (NO in step S205), the recording unit 203 records the acquired image as image data in association with the recognized patient number in the storage unit 204 (step S206). Then, the process goes back to step S205.

### «Image Data Storage Process»

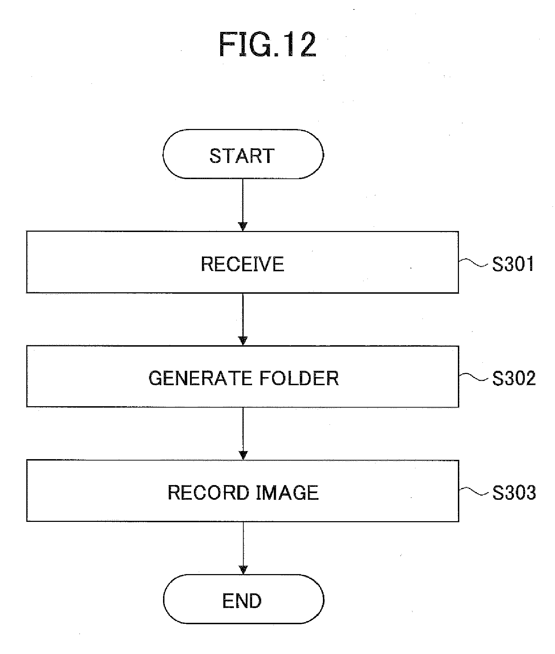

FIG. 12 is a flowchart illustrating an image data storage process executed by the information processing apparatus 100 according to an embodiment of the present invention.

First, the image receiving unit 102 receives a combination of a patient number and image data transmitted from the image transmitting unit 205 of the image capturing apparatus 200 (step S301). The recording unit 103 generates a folder in the storage unit 101 using the patient number received by the image receiving unit 102 (step S302). Then, the recording unit 103 records the image data 151 associated with the patient number in the generated folder (step S303). FIG. 9 illustrates an exemplary screen showing folders generated by the recording unit 103 and image data stored therein.

### «Specific Operation Example»

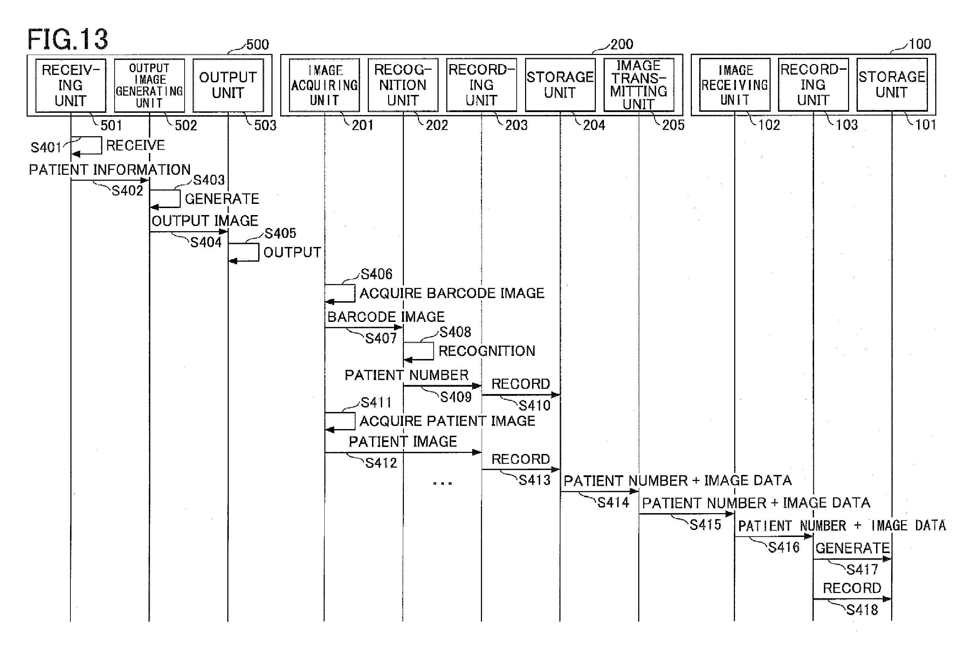

FIG. 13 is a sequence chart illustrating a specific operation example of the image management system 1 according to an embodiment of the present invention. FIG. 13 illustrates an exemplary operation sequence that involves printing (outputting) patient information on the sheet 4 as illustrated in FIG. 7 and acquiring an image of a patient using the barcode printed on the prefabricated wristband included in the sheet 4.

First, the receiving unit 501 of the image forming apparatus 500 receives patient information including a patient number (e.g., "1234567890"), a patient name (e.g., "patient A"), and a patient photo, for example, from the management system 300 (step S401). Then, the output image generating unit 502 receives the patient information from the receiving unit 501 (step S402). Then, the output image generating unit 502 generates a barcode corresponding to the patient number "1234567890" and generates an output image for printing including the barcode and some or all of the patient information as illustrated in FIG. 7, for example (step S403).

The output image generating unit 502 passes the generated output image to the output unit 503 (step S404). The output unit 503 outputs the output image received from the output image generating unit 502 and outputs (prints) the output image onto the sheet 4 (step S405).

Then, medical personnel may hand the prefabricated wristband with the barcode that can be detached and assembled from the sheet 4 to the patient, and read the barcode printed on the wristband using the image capturing apparatus 200.

The image acquiring unit 201 of the image capturing apparatus 200 acquires an image of the barcode that is printed on the wristband (step S406). Then, the image acquiring unit 201 passes the acquired barcode image to the recognition unit 202 (step S407). Then, the recognition unit 202 recognizes the patient number "1234567890" from the barcode image (step S408). The recognition unit 202 passes the recognized patient number to the recording unit 203 (step S409). The recording unit 203 records the received patient number "1234567890" in the storage unit 204 (step S410).

Then, medical personnel can capture an image of an affected area of the patient using the image capturing apparatus 200.

The image acquiring unit 201 of the image capturing apparatus 200 acquires an image of the affected area of the patient (step S411). Then, the image acquiring unit 201 passes the acquired image of the affected area of the patient to the recording unit 203 (step S412). The recording unit 203 records the received image of the affected area of the patient in association with the corresponding patient number "1234567890" recorded in step S410 in the storage unit 204 (step S413).

Note that the medical personnel may capture images of the patient multiple times. In this case, the image capturing apparatus 200 repeatedly executes the above-described process steps S411-S413. Then, the medical personnel may connect the image capturing apparatus 200 to the information processing apparatus 100 by a USB cable, for example.

The image transmitting unit 205 of the image capturing apparatus 200 reads the combination of the patient number and the image data from the storage unit 204 (step S414). The image transmitting unit 205 transmits the acquired combination of the patient number and the image data to the information processing apparatus 100 (step S415).

The image receiving unit 106 of the information processing apparatus 100 passes the received combination of the patient number and the image data to the recording unit 107 (step S416). The recording unit 107 generates a folder with the folder name "1234567890" corresponding to the received patient number in the storage unit 101 (step S417). Thereafter, the recording unit 107 records the received image data in the generated folder "1234567890" (step S418). FIG. 9 illustrates an example in which four sets of image data are stored in the folder "1234567890".

In the above example, the image capturing apparatus 200 has been described as acquiring an image of the affected area of the patient. Note that in some embodiments the image capturing apparatus 200 may acquire a moving image of the patient, for example. In this case, the recording unit 204 of the image capturing apparatus 200 may store a combination of the patient number and the moving image data in the storage unit 204, for example. Alternatively, the recording unit 204 may store the patient number in header information of the moving image data, for example.

Also, in the above example, the combination of the patient number and the image data read from the barcode is transmitted from the image capturing apparatus 200 to the information processing apparatus 100. However, note that in the case where the patient number is stored in the header information of the image data as described above, only the image data is actually transmitted from the image capturing apparatus 200 to the information processing apparatus 100.

Also, in the above example, the image capturing apparatus 200 is connected to the information processing apparatus 100 by the medical personnel using a USB cable. Note, however, that in some embodiments, the image capturing apparatus 200 and the information processing apparatus 100 may be configured to establish communication via a wireless LAN, for example. In this case, the image capturing apparatus 200 may be configured to automatically transmit image data to the information processing apparatus 100 when images of a certain number of patients have been acquired, for example.

As described above, according to an aspect of the image management system 1 of the present embodiment, medical personnel may be able to associate a patient and image data of the patient using the image capturing apparatus 200 and the information processing apparatus 100 without performing a special operation. In this way, a mismatch between the patient and the image data due to operational errors by the medical personnel may be prevented, for example. Further, the need to operate electronic devices while engaging in medical practice may be minimized such that issues related to hygiene may be reduced, for example.

### «Applications»

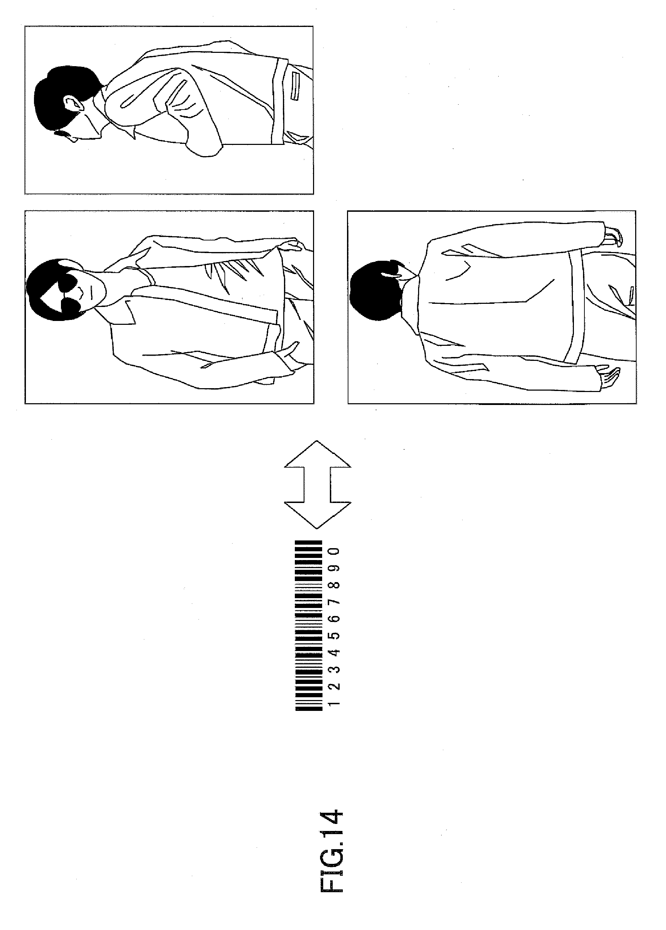

In the following, exemplary applications of the image management system 1 to non-medical systems are described with reference to FIGS. 14-17.

### «Clothing Inventory Management System»

FIG. 14 illustrates an exemplary application of the image management system 1 to a clothing inventory management system. The illustrated clothing inventory management system manages an identification number assigned to a clothing item and image data of one or more views of the clothing item. In the present example, the image management system 1 as described above may be used to manage image data of one or more images of an imaging object in association with one identification number.

That is, a seller of clothing may assign an identification number to a product in advance and attach a barcode representing the corresponding identification number to the product. Then, according to the operations described above, the image capturing apparatus 200 may read the barcode and capture one or more images of the product thereafter. In this way, the information processing apparatus 100 may store image data of one or more images of a product in a folder that is individually provided for the corresponding identification number of the product.

Thus, the seller of clothing may be relieved of the need to manually associate the image data with the corresponding identification number, and the burden on the seller may be reduced as a result.

### «Auction Product Management System»

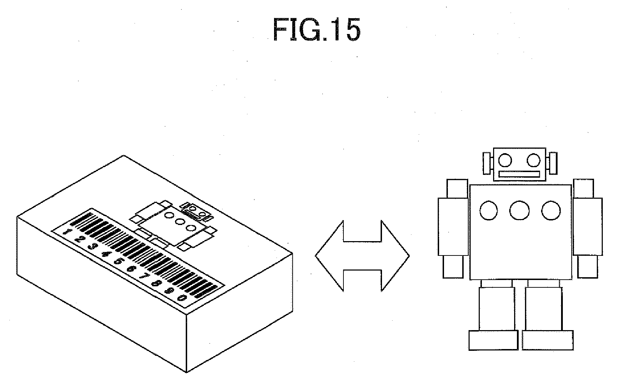

FIG. 15 illustrates an exemplary application of the image management system 1 to an Internet auction product management system. The illustrated Internet auction product management system manages an identification number attached to a box for a product (a plastic model is illustrated in the present example) and image data of one or more views of the product. As in the clothing inventory management system illustrated in FIG. 14, in the present example, the image management system 1 as described above may be used to manage image data of one or more images of an imaging object in association with one identification number.

That is, an Internet auction seller may assign an identification number to a product in advance and attach a barcode representing the corresponding identification number to a box for the product. Then, according to the operations described above, the image capturing apparatus 200 may read the barcode and capture one or more images of the product thereafter. In this way, the information processing apparatus 100 may store image data of one or more images of a product in a folder that is individually provided for the corresponding identification number of the product.

Thus, the Internet auction seller may be relieved of the need to manually associate the image data with the corresponding identification number, and the burden on the seller may be reduced as a result.

### «Insurance Company Accident Record Management System»

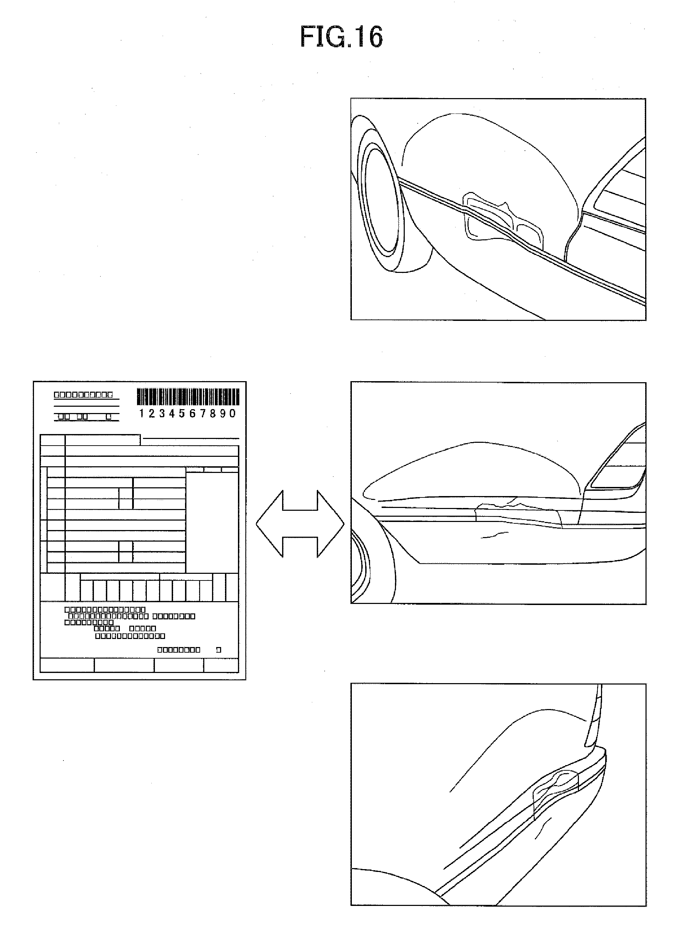

FIG. 16 illustrates an exemplary application of the image management system 1 to an insurance company accident record management system for managing of accident records. For example, an insurance company may manage image data of one or more images related to an accident (e.g., images of an accident vehicle) in association with one identification number assigned to the accident. In the present example, the above-described image management system 1 may be used to manage image data of one or more images related to an event (accident) in association with one identification number.

That is, according to the above-described operations, an insurance company representative may first use the image capturing apparatus 200 to read a barcode included in a document for keeping records of an accident and then capture images of a vehicle that was involved in the accident. In this way, the information processing apparatus 100 may store image data of one or more images of an accident vehicle in a folder that is individually provided for the corresponding identification number of the accident. Thus, the insurance company representative may be relieved of the need to manually associate the image data with the corresponding identification number.

### «School Work Record Management System»

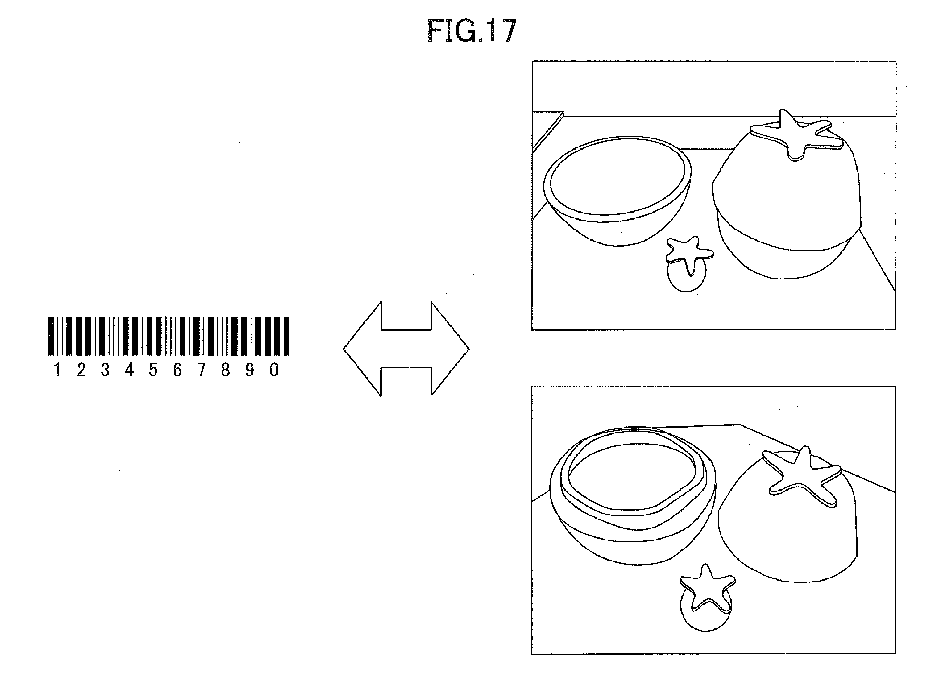

FIG. 17 illustrates an exemplary application of the image management system 1 to a work record management system used in an educational institution such as a school. For example, an elementary school teacher may manage image data of a work created by a student in association with an identification number assigned to the student. In the present example, the image management system 1 described above may be used to manage image data of one or more images of an imaging object in association with one identification number.

That is, according to the operations described above, the elementary school teacher may first use the image capturing apparatus 200 to read a barcode representing the identification number assigned to a student and then capture one or more images of the work created by the student. In this way, the information processing apparatus 100 may store image data of one or more images of a work in a folder that is individually provided for the corresponding identification number of the student.

### «Modification»

In the above-described embodiments, the storage unit 101 of the information processing apparatus 100 may be configured to store a usage period (lease period) of the image capturing apparatus 200 that is leased to a client, for example. In turn, the transmitting unit 104 of the information processing apparatus 100 may convey to the client via email, for example, corresponding usage fee information indicating a usage fee calculated based on the usage period. Also, the storage unit 101 of the information processing apparatus 100 may be configured to store the total number of barcodes output (printed out) by the image forming apparatus 500. In turn, the transmitting unit 104 of the information processing apparatus 100 may convey to the client via email, for example, usage fee information indicating a usage fee calculated based on the total number of barcodes output, for example.

Although the present invention has been described above with reference to certain illustrative embodiments, the present invention is not limited to these embodiments, and numerous variations and modifications may be made without departing from the scope of the present invention.

The present application is based on and claims the benefit of priority of Japanese Patent Application No. 2014-102504 filed on May 16, 2014, the entire contents of which are hereby incorporated by reference.

## Claims

1. An image management system (1) comprising:
an image capturing apparatus (200); and
an information processing apparatus (100);
wherein the image capturing apparatus includes
a recognition unit (202) configured to recognize identification information that is represented by an optically readable code;
an acquiring unit (201) configured to acquire image data of at least one image of an imaging object;
a recording unit (203) configured to record the identification information recognized by the recognition unit and the image data acquired by the acquiring unit in association with each other; and
a transmitting unit (205) configured to transmit the identification information and the image data recorded by the recording unit to the information processing apparatus; and
wherein the information processing apparatus includes a storage unit (101) configured to store the image data based on the identification information received from the image capturing apparatus.

2. The image management system as claimed in claim 1, wherein the storage unit includes a folder corresponding to the identification information and is configured to store the image data associated with the identification information in the folder.

3. The image management system as claimed in claim 1, wherein the identification information includes a name and a number for identifying the imaging object.

4. The image management system as claimed in claim 3, wherein
the recognition unit recognizes the identification information from the optically readable code that is printed on an accessory carried by a patient corresponding to the imaging object; and
the recording unit records the image data including at least one of a partial body image and a whole body image of the patient acquired after the identification information has been recognized by the recognition unit in association with the recognized identification information.

5. The image management system as claimed in claim 4, wherein the identification information includes a name and an identification number of the patient.

6. The image management system as claimed in claim 3, wherein
the recognition unit recognizes information identifying a name of a product or a creator of a work as the identification information represented by the optically readable code; and
the recording unit records the image data including an image of at least one part of the product or the work acquired after the identification information has been recognized by the recognition unit in association with the recognized identification information.

7. The image management system as claimed in claim 3, wherein
the recognition unit recognizes information identifying an event as the identification information represented by the optically readable code; and
the recording unit records the image data including an image related to the event acquired after the identification information has been recognized by the recognition unit in association with the recognized identification information.

8. The image management system as claimed in claim 1, wherein the information processing apparatus generates usage fee information based on a usage period of the image capturing apparatus.

9. The image management system as claimed in claim 1, wherein the information processing apparatus generates usage fee information based on an output number of the optically readable code.

10. An image management method that is implemented by an image management system (1) including an image capturing apparatus (200) and an information processing apparatus (100), the image management method comprising:
a recognition step (S408) of recognizing identification information that is represented by an optically readable code;
an acquisition step (S411) of acquiring image data of at least one image of an imaging object;
a recording step (S413) of recording the identification information recognized in the recognition step and the image data acquired in the acquisition step in association with each other;
a transmission step (S415) of transmitting the identification information and the image data recorded in the recording step to the information processing apparatus; and
a storage step (S418) of storing the image data based on the identification information received from the image capturing apparatus.
